# EUROPEAN PATENT SPECIFICATION

(11) **EP 2 457 575 B1**
(45) Date of publication and mention of the grant of the patent: **16.03.2016**
(21) Application number: 10460046.5
(22) Date of filing: 29.11.2010
(51) Int. Cl.: A61K 35/747, C12R 1/225, A61P 31/04

(54) **New method for obtaining a Lactobacillus reuteri strain useful in medical and veterinary prophylaxis and treatment**
Neues Verfahren zur Herstellung eines Lactobacillus reuteri Stammes verwendbar in der medizinischen und tiermedizinischen Prophylaxe und Behandlung
Nouveau procédé permettant d'obtenir une nouvelle souche de Lactobacillus reuteri utile dans la prophylaxie et le traitement médical et vétérinaire

(43) Date of publication of application: 30.05.2012
(73) Proprietor: Eurochit Danuta Kruszewska, 02-765 Warszawa (PL)
(72) Inventor: Kruszewska, Danuta, 02-765 Warszawa (PL)
(74) Representative: Malewska, Ewa

(56) References cited:
- DATABASE WPI Week 200414 Thomson Scientific, London, GB; AN 2004-140523 XP002635004, & KR 2003 064 462 A (BIOTOPIA CO LTD) 2 August 2003 (2003-08-02)

## Description

The present invention relates to a new method for obtaining a nanoproduct useful in prophylaxis and medicine, both human and veterinary. A new microorganism isolated and identified by the present inventor - Danuta Kruszewska (DK), is a source of that product.

Throughout the lifetime of a healthy human (as well as other animals), microorganisms are present in his/her organism. Among these microorganisms, bacteria prevail, although fungi and protozoa are also present. Various types of microorganisms colonize most intensively the mucosa of the gastrointestinal tract, body surface and other systems, e.g. urogenital or upper airways, and settle on mucosal surfaces. The mechanisms, according to which individual species/strains of a specific microbiota, as well as the microbiota as a whole perform their function in the maintenance of health, have not been fully recognized. Microbiota of the gastrointestinal tract, the skin, and other ecological niches inhabited by microbes differ with respect to the functions performed, composition and amount of microorganisms. Thus, the term 'microbiota' is to be understood as referring to a 'herd' of microorganisms inhabiting in a specific anatomical and physiological region of the body, which constitutes a microbiom of the microorganisms.

Mutual interactions of the gastrointestinal tract microorganisms have the character of a symbiotic co-existence. Based on the example of the alimentary system, the microbiota of the system stimulate host immunity, not only on the level of a microorganism (live or dead), but also through its intra- and extra-cellular metabolites, counteract the anchoring of other microorganisms dangerous for health (mainly pathogens) - through competitiveness with respect to receptor and substrate, neutralize toxic intra- and extra-cellular metabolites of other microbes, regulate the development and physiological functions of the intestine (not only on the level of digestive functions), ferment indigestible, but energetically useful substrates, metabolize glycans and amino acids, and synthetize vitamins.

*Helicobacter pylori* is a microorganism colonizing gastric and duodenal mucous membranes, responsible for a number of medical conditions, including gastritis and other related diseases, such as gastric and duodenal ulcer, stomach (gastric) cancer, duodenal cancer, intestinal disorders, disorders of the gastrointestinal tract (GIT) and diarrhoea which, in fact, afflict every representative of the human species living in the modern societies of Western countries. This is the main medical problem for 1/3 of the human population, which has growing medical, social and economic consequences. Over 10 million Americans suffer due to *H. pylori*-related gastritis; although applying more recent definitions of gastritis the number of afflicted patients is estimated to be 14-25 million, including those who have not yet experienced gastric ulcer or cancer, but already experience sufficiently positive pain symptoms. Despite the known role *of H. pylori* in the pathogenesis of gastritis, the development of peptic ulcer and adenocarcinoma (see: Hunt RH. The role of Helicobacter pylori in pathogenesis: the spectrum of clinical outcomes. Scand J Gastroenterol Supl. 1996; 220: 3-9), it is also suggested that certain strains of *H. pylori* may occur in nature as commensals (see: Misiewicz J. Is the only good Helicobacter a dead Helicobacter? Helicobacter 1997: 2S: S89-S91).

The National Institute of Health of the United States of America estimates that in 1990 the cost of treatment of the above-mentioned gastritis and other related diseases, e.g. gastric and duodenal ulcer, stomach (gastric) cancer, duodenal cancer, intestinal disorders, GIT disorders and diarrhoea, reached $20 billion. With the ageing of the population and an increasing prevalence of gastritis, in the Unites States alone it is anticipated that up-till 2020 just the medical costs associated with these medical conditions will be over $60 billion.

The current methods of treatment and prophylaxis of gastritis and other related diseases, such as the above-mentioned gastric and duodenal ulcer, gastric cancer, duodenal cancer, intestinal disorders, GIT disorders, and diarrhoea, the development of which is *H. pylori* related, are usually based on decreasing the HCl production in stomach, or killing generally all the bacteria with antibiotics. Table 1 shows the effectiveness of the to-date methods of treatment.

**Table 1. Known and commonly applied therapeutic strategies for the treatment of gastritis.**

| Strategy | Antibiotics | H⁺(protons) inhibitors |
|---|---|---|
| Reduction of stomach colonization by *H. pylori* | Elimination | Poor effect or lack of effect |
| Modulation of HCl secretion | Lack of effect | Reduction of gastric secretion |

In the alimentary system there also occur beneficial lactic acid bacteria (LAB) which, as such, as well as their products, are considered a part of natural health ameliorating microbiota and as agents improving the health of the host and the intestine (see: Kullisaar T, Zilmer M, Mikelsaar M, Vihalemm T, Annuk H, Kairane C, et al. Two antioxidative lactobacili strains as promising probioticts. Int J Food Microbiol 2002; 72: 215-24).

LAB are generally recognised as safe (GRAS) bacteria. Despite the outstanding effect on the general health status, especially the effect on the GIT, LAB bacteria show an insufficient capability of GIT colonization after the period of breast suckling in mammals, and generally, for the whole life-time in birds. This is due, firstly, by the deficiency of suitable substrates for the growth of LAB after the period of breast suckling, and secondly - characteristic of bacterial wall which is incapable of producing fimbriae, etc., responsible for the bacterial adhesion to the GIT epithelium or to the GIT mucosa.

Antibiosis between LAB and *H. pylori* is rarely employed as a therapy, which is due to the low efficiency of such a treatment because LAB cannot easily colonize the gastric mucosa.

Many LAB are currently available in commerce, offered, as live cultures, food additives for humans and feed additives for animals, such as for example, *Lactobacillus plantarum* 299 v, and others. It is expected that the products of LAB growing in the alimentary system will be beneficial for the consumer of these bacteria. This is very often controversial because live LAB cultures are excellently eliminated and killed by antibacterial compounds of the host, which basically reduce the growth of bacteria in stomach and the upper part of intestine.

Various other medical disorders may also secondarily lead to the development of gastritis. Such medical disorders are presented in Table 2.

**Table 2. Medical conditions and methods of treatment causing gastritis or other related diseases, e.g. gastric and duodenal ulcer, gastric cancer, duodenal cancer, intestinal disorders, GIT disorders and diarrhoea.**

| Classical diseases | Drug, drug abuse, allergy |
|---|---|
| Type 1 diabetes | NSAID (non-steroid anti-inflammatory drugs) |
| Type 2 diabetes | Methotrexate |
| Inflammatory bowel disease | Antibiotics |
| Lymphoma | Excess of thyroid hormone |
| Primary billiary cirrhosis | Alcohol |
| Rheumatoid arthritis | Immobilization |
| Coeliac disease | Hypovitaminosis |

In mammals and chickens the proper functioning of stomach is a problem. Especially in the case of force-fed fast growing animals, with feeding a very non-physiological feed, the stomach does not develop correctly and many health disorders occur, the problems caused by various forms of gastritis, which is the cause of decreased efficiency of animal production. In order to prevent such conditions, which not only cause unnecessary suffering to animals, but also increase farmers' costs, a better understanding and control of physiological and pathological processes in the gastric mucosa - associated with the presence of *H. pylori* and other pathogens of the gastrointestinal tract, is necessary in the postnatal life of vertebrates, including mammals and birds.

In the light of the above indicated, known in the art inconveniences of the to-date therapies, prevention and alleviation of gastric diseases and related conditions, as well as the high costs of treatment and correction of GIT function in conditions related to, for example, gastritis or gastric ulcer, it is the aim of the presented invention to provide a new product and improved methods and compositions for use in human medicine in order to improve functioning of the stomach by elimination or regulation of the growth of *H. pylori* and other pathogens of the gastrointestinal tract.

Further aim of the invention is to provide a veterinary product and method for amelioration of the function of the stomach and remaining parts of the GIT in vertebrates, especially in mammals and birds, by means of elimination or regulation of the growth of *H. pylori* and other pathogens of the gastrointestinal tract.

It is also the aim of the invention to provide a new antibacterial and/or antimicrobial agent for use in prophylaxis and treatment of medical conditions developing as a result of infections induced by bacteria, fungi and other pathogens of the alimentary system, body surface, and other systems, such as the urogenital and respiratory systems in vertebrate, including human, other mammal and bird.

The above-mentioned and other goals were achieved due to the development of a solution based on the isolation and identification of a new microorganism - *Lactobacillus reuteri* DAN080, deposited on June 20, 2003 - in accordance with a Budapest Treaty on the International Recognition of the Deposit of Microorganisms for the Purposes of Patent Procedure, in DSMZ collection - Deutsche Sammlung von Mikroorganismen und Zellkulturen GmbH, in Braunschweig, DE, access number: DSM 15693.

The present invention concerns *Lactobacillus reuteri* DAN080 strain with deposit number DSM 15693 for use in medicine.

The invention relates to cultures of *L. reuteri* DAN080, the partially inactivated cultures of *L. reuteri* DAN080, liquid supernatants of *L. reuteri* DAN080 cultures, concentrated supernatants of *L. reuteri* DAN080 cultures, and dried supernatants of *L. reuteri* DAN080 cultures, for use as an antimicrobial agent in prophylaxis and treatment of medical conditions developed as an effect of infections caused by bacteria, fungi and other pathogens of the gastrointestinal tract, body surface, and other systems, such as the urogenital system, respiratory system in vertebrates.

According to the invention the *Lactobacillus reuteri* DAN080 strain with deposit number DSM 15693 is used in a form selected from a group comprising the whole culture of *L. reuteri* DAN080, liquid supernatant, concentrated supernatant, and dried supernatant obtained from the culture of *L. reuteri* DAN080, and from mixed bacterial cultures comprising *L. reuteri* DAN080, or mixtures thereof for prophylaxis and treatment of medical conditions developed as an effect of infections caused by bacteria, fungi and other pathogens of the gastrointestinal tract, body surface, and other systems, such as the urogenital system, respiratory system in vertebrates.

According to the invention the vertebrate is human individual.

The vertebrate is also a domestic animal, pet, animal involved in sport, broiler, layer hen, mouse, rat, guinea pig, rabbit and other laboratory animal, including primates, independently of their age.

According to the invention the microorganism is a pathogenic bacterium or fungi.

In particular, a pathogenic bacterium is *H. pylori.*

The invention relates also the use of the *Lactobacillus reuteri* DAN080 strain with deposit number DSM 15693 of the present invention for manufacturing a composition for treatment, alleviation or prophylaxis of GIT disorders, gastritis, gastric ulcer, duodenal ulcer, intestinal disorders and diarrhoea in individuals in need of such a treatment, and being vertebrates including humans, other mammals and birds, which composition comprise the agent in an effective amount, that provides for obtaining the desired preventive or therapeutic effect.

According to the invention the composition is in particular to kill, inhibit, regulate, and prevent the growth of *H. pylori* and other microorganisms, and to be administered in an effective amount and at a sufficient rate, necessary for reaching the desired preventive or therapeutic result.

In particular, the composition is a pharmaceutical composition comprising optionally, other biologically active substances, such as vitamins, especially vitamins D and E, especially in a nanoform, salts of lactic acid and other acids comprised in the *L. reuteri* DAN080 metabolites, in preventive or therapeutic doses, and pharmaceutically acceptable carriers and/or additions.

Preferably, the pharmaceutical composition is in a solid form and is divided into single doses comprising therapeutically effective amount of the present antimicrobial agent, in the amount of from 0.001 to 0.2 g/kg of body weight per day. The composition has in particular a form of a tablet or capsule.

Alternatively, the pharmaceutical composition of the present invention is in a liquid form and is divided into single doses comprising a therapeutically effective amount of the present antimicrobial agent, in the amount of from 0.001 to 0.2 g/kg of body weight per day, especially in an ampoule. Such composition has a liquid form for use as aerosols, cataplasm or moist compress.

Preferably, in another aspect of the invention, the composition is a dietary supplement, food or beverage. The dietary supplement, food or beverage is in a solid form and/or in form of beverage. Preferable, therapeutically effective amount of the antimicrobial agent of the present invention is from 0.001 to 0.2 g/kg of body weight per day.

Especially preferably, a product of fermentation of *Lactobacillus reuteri* DAN080 may be used in form of cultures, at least partially inactivated cultures and the supernatants of these cultures - respectively processed, for the modulation of the function of stomach, intestine, and GIT in vertebrates in need for such treatment, including humans, other mammals and birds.

Although the present invention is discussed with reference to the exemplary influence exerted on *H. pylori -* as a pathogenic agent present in the human gastrointestinal tract, when administered *per os,* the demonstrated biological activity of live cultures of the new bacteria *L. reuteri* DAN080 and the above mentioned derived forms, allows those skilled in the art to use the solution according to the invention in the prophylaxis and treatment of pathological conditions developed under the influence of other pathogens in other systems of the human body and those of other mammals and birds, and with different routes of administration.

Further goals and advantages resulting from the present invention will be discussed below in the detailed description of the invention, with the reference to the drawing, wherein:
Fig. 1 - presents in graphic form the results of plate diffusion assay (agar medium GAB-CAMP with the strain *H. pylori* 17874): 1 - non-active supernatant obtained from the culture of *L. reuteri*; 2 - non-active broth MRS; 3 and 4 - inhibition zones caused by the activity of the active supernatant obtained from the culture *of L. reuteri* DAN080;
Fig. 2 - illustrates the effect of supernatant from the culture of *L. reuteri* DAN080 on the growth of *H. pylori* in a liquid medium - BHI broth;

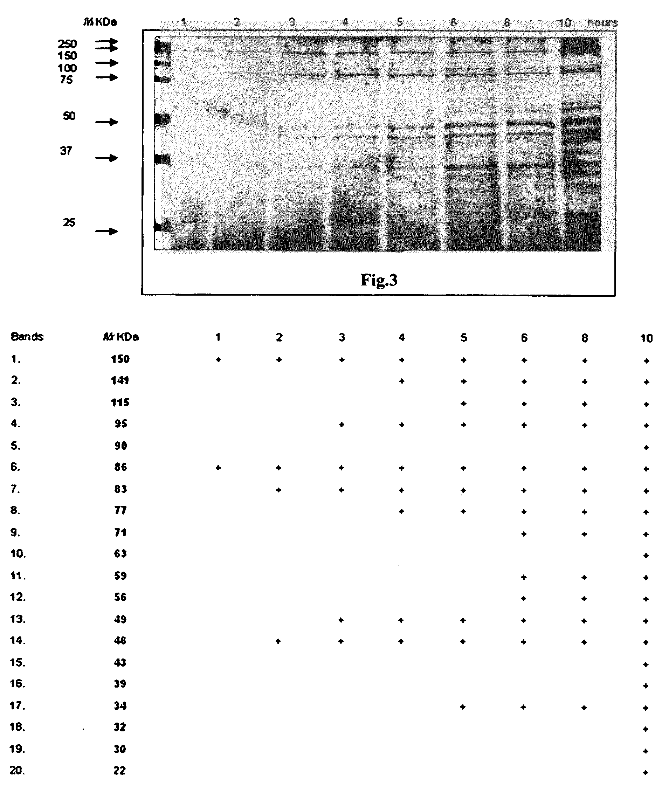

Fig. 3 - presents SDS-PAGE electrophoresis of supernatants from *L. reuteri* DAN080 obtained, respectively, after 1, 2, 3, 4, 5, 6, 8 and 10 hours of growth in MRS broth. Numbers 1 - 20 designate the identified proteins released into the medium by *L*. *reuteri*;
Fig. 4 - illustrates the identification of *L. reuteri* DAN080 by Denaturating Gradient Gel Electrophoresis *L. reuteri* DAN080 PCR product amplified with primers LacF and LacR.
Fig. 5 - shows the relationship between the activity of lysozyme (U/L) in rat blood, and the presence of the bacteria *L. reuteri* DAN080 in their gastrointestinal tracts, following intragastric administration.
Fig. 6 - presents the relationship between the activity of neurons isolated from the Enteric Nervous System and extracellular metabolites *of L. reuteri* DAN080

### Detailed description of the invention

Bacteria *L. reuteri* DAN080 were isolated from the gastrointestinal tract of a healthy laboratory animal. The bacteria were isolated as a single colony on a solid medium with blood agar. This medium was incubated with the scrapings from the gastrointestinal tract of a healthy mouse at the temperature of 37°C for 24 hours. The colony isolated was proliferated in the broth MRSB (Oxoid), on a standard medium for lactic acid bacteria (LAB). The pH of the medium prior to sterilization was pH 6.8. Sterilization was performed within 15 min., at temp. of 121°C, the pH of the medium after sterilization: pH 6.2. The thermal conditions of bacteria incubation remained within the range 35±3°C. The full growth in the liquid culture was 16 hours. The bacteria can be stored, with warranty of survival at the temperature of -20°C. *L. reuteri* DAN080, survive at room temperature +20 to +22°C for at least 30 days maintaining the capability of inhibiting the growth of other microorganisms.

In order to obtain an enhanced antimicrobial activity the bacteria *L. reuteri* DAN080 are grown on media comprising AKG.

Composition of the medium: 0.5% meat extract ; 0.5% yeast extract; 1% peptone; 0.3 % NH₄Cl; 0.4 % K₂HPO₄; 0.4 % KH₂PO₄; 0.01% MgSO₄ x 7 H₂O; 0.005 % MnSO₄ x 4 H₂O; 0.1% Tween 80; 0,05 L-cysteine HCl; 0.0002 of each of the following vitamins: B1, B2, B6, B5, B12, B9. After sterilization in the conditions as presented above, 23 mmol/l maltose and, respectively, starch or glucose, and 10 mmol/l AKG were added to the medium. The medium pH was: pH 6.2. The bacteria were incubated at 37°C for 16 hours. An enhanced bacterial growth occured in the presence of AKG. In the medium, from 4 to 6 mmol/l of acetates and lactates were found, while in the medium non-enriched with AKG - from 1 to 2 mmol/l.

*Lactobacillus reuteri* DAN080 was identified in accordance with biochemical activity, where the capability of fermenting carbohydrates was assessed by the test - Api 50 CH and CHL medium, bioMerieux SA, Marcy lÉtoile, France.
Taxon: *L. reuteri*
- phenotypic characteristics according to the API system
CAT: 1053 1121 0000 000 000 0000000
API RID32s: 515 151 511 111 315 111 111 511 111 111 11 1
API 50CHL: 1111533111 5511111111 1411111155 5511151111 1111111311
Api ID32AN: 155 515 111 114 111 513 351 351111 312

Genotypic characteristics [SEQ. ID NO: 1, 2, 3]: based on DNA analysis and comparison with 16S gene sequence of rhibosomal RNA *L. reuteri* (see: GenBank: EF187261.2; Byun R, Nadkarni MA, Chhour KL, Martin FE, Jacques NA, Hunter N. Quantitative analysis of diverse Lactobacillus species present in advanced dental caries. J Clin Microbiol. 2004;42(7):3128-36; Fredricks DN, Relman DA. Improved amplification of microbial DNA from blood cultures by removal of the PCR inhibitor sodium polyanetholesulfonate. J Clin Microbiol. 1998; 36(10):2810-6). The following primers were useful for sequencing [SEQ. ID NO: 2, 3] - primer 1: TGGAAACAGA TGCTAATACC GC (22 bp) [SEQ. ID NO: 2], primer 2: ATTAGATACC CTGGTAGTCC (20 bp) [SEQ. ID NO: 3]

tggaaacaga tgctaatacc gcataacaac aaaagccaca tggcttttgt
ttgaaagatg gctttagcta tcactctggg atggacctgc ggtgcattag
ctagttggta aggtaacggc ttaccaaggc gatgatgcat agccgagttg

agagactgat cggccacaat ggaactgaga cacggtccat actcctacgg

gaggcagcag tagggaatct tccacaatgg gcgcaagcct gatggagcaa

caccgcgtga gtgaagaagg gtttcggctc gtaaagctct gttgttggag

aagaacgtgc gtgagagtaa ctgttcacgc agtgacggta tccaaccaga

aagtcacggc taactacgtg ccagcagccg cggtaatacg taggtggcaa

gcgttatccg gatttattgg gcgtaaagcg agcgcaggcg gttgcttagg

tctgatgtga aagccttcgg cttaaccgaa gaagtgcatc ggaaaccggg

ccacttgagt gcagaagagg acagtggaac tccatgtgta gcggtggaat

gcgtagatat atggaagaac accagtggcg aaggcggctg tctggtctgc

aactgacgct gaggctcgaa agcatgggta gcgaacagga ttagataccc

tggtagtcc [SEQ. ID NO: 1] (659 bp)

After a specified time of *L. reuteri* DAN080 growth, the culture is centrifuged and the liquid supernatant, concentrated supernatant, and dried or lyophilized supernatant is the product of a specific capability and activity for regulating the growth of *H. pylori* and other bacteria *in vitro* and *in vivo.* After electrophoretic separation performed from the liquid supernatant, the concentrated supernatant and dried supernatant collected at a predetermined time, specific proteins of a molecular weight within the range 150 - 22 kD or less were visualized, which proteins are responsible for homeostasis and regulation of the growth of *H. pylori.* These bands are weak, and do not occur in electrophoregraphs obtained from liquid supernatant, concentrated supernatant and dried supernatant harvested at a time different than the above-specified time for *L. reuteri* DAN080 culture, and the liquid supernatant, concentrated supernatant and dried supernatant, show one of the proper effects - homeostatic and growth regulating, on *H. pylori* and other bacteria *in vitro,* as well as *in vivo.*

For the genetic identification of *L. reuteri,* the total genomic DNA was isolated from bacteria cultured overnight, with the use of the kits DNaesy™, Qiagen GmbH, Hilden, Germany.

Amplification of the 340 bp fragment of 16S rDNA was performed with semi-nested PCR (first run: 94°C for 30 s., 61°C for 60 s., 68°C for 60 s., 35 cycles; second run: 94°C for 30 s., 58°C for 60 s., 68°C for 60 s., 40 cycles) using primers [SEQ. ID NO: 4, 5, 6] (see: Walter, J., Hertel, Ch., Tannock GW., Lis CM., Munro K., Hammes W.P. (2001) Detection of Lactobacillus, Pediococcus, Leuconostoc i Weisella species in human faeces by using group specific PCR primers and denaturing gradient gel electrophoresis. Appl. Environ. Microb. 67, 2578-2585); forward primer 3: AGCAGTAGGG AATCTTCCA (19 bp) [SEQ. ID NO: 4]; reverse primer 4: ATTYCACCGC TACACATG (18 bp) [SEQ. ID NO: 5]; forward primer 5: ACAATGGACG AAAGTCTGAG TG (22 bp) [SEQ. ID NO: 6].

### Definitions

As used herein, the term 'killing, inhibiting, regulating, preventing growth of *H. pylori* and other bacteria product', is intended to mean the pharmacological, chemical, mechanical and physiological characteristics of *L. reuteri* DAN080 cultures, partially inactivated *L. reuteri* DAN080 cultures, liquid supernatants *of L. reuteri* DAN080 cultures, condensed supernatants *of L. reuteri* DAN080 cultures and dried supernatants *of L. reuteri* DAN080 cultures, as measured by means of certain parameters applied in accordance with the invention. Such parameters are known to those skilled in the art, and are further defined in the presented description.

As used herein, the term 'improvement in gastritis and other related diseases, e.g. gastric and duodenal ulcer, intestinal disorders, GIT disorders, and diarrhoea by the elimination or stabilization of *H. pylori* growth' is intended to mean the chemical and physiological characteristics of the stomach, intestine, and GIT, as measured by means of certain parameters applied in accordance with the invention. Such parameters are known to those skilled in the art and are further defined in the presented description.

The term 'improvement in gastritis and other related diseases, e.g. gastric and duodenal ulcer, intestinal disorders, GIT disorders, and diarrhoea by the elimination or stabilization of *H. pylori* growth', is intended to additionally mean, in the present description, changes in mechanical, chemical and physiological characteristics of stomach, intestine and GIT functioning, thus defining the quality of the stomach, intestine and GIT as compared to those of vertebrates, including mammals and birds, with respect to which no prophylaxis and/or treatment is applied, or which are not administered in accordance with the present invention, with any culture of *L. reuteri* DAN080, partially inactivated *L. reuteri* DAN080 culture, liquid supernatant *of L. reuteri* DAN080 culture, concentrated supernatant of *L. reuteri* DAN080 culture and dried supernatant *of L. reuteri* DAN080 culture. The changes are considered as an improvement if such changes are positive for vertebrates, including mammals and birds.

The term 'improvement in gastritis and other related diseases, e.g. gastric and duodenal ulcer, intestinal disorders, GIT disorders, and diarrhoea by the elimination or stabilization of *H. pylori* growth', as intended in the present description may also mean a change, modification or other effect on the current mechanical, chemical and physiological characteristics of the stomach, intestine, GIT and colonization of the same by *H. pylori.*

According to the meaning defined in the present description and claims, the term 'pharmaceutical composition' means therapeutically and/or preventively effective composition of the invention comprising *L. reuteri* DAN080 cultures, partially inactivated *L. reuteri* DAN080 cultures of, liquid supernatants of *L. reuteri* DAN080 cultures, concentrated supernatants of *L. reuteri* DAN080 culture and dried supernatants of *L. reuteri* DAN080 cultures.

The term 'therapeutically effective amount' or 'effective amount' or 'therapeutically effective' applied in the presented description and claims refers to the amount of antimicrobial agent of the invention, that provides the therapeutic and/or preventive result when used in a specific condition and a specific administration regime. The term means a predetermined amount of the active material calculated so as to produce the desired therapeutic and/or preventive effect. The above-mentioned active material may be combined with an appropriate additive, for example, other microorganisms or diluent, or carrier or administration vehicle. In addition, the term is intended to mean an amount sufficient to reduce, and most preferably to prevent a clinically significant deficiency in vertebrates in need of such treatment, the vertebrates including mammals and birds. The establishment of a therapeutically effective amount remains within the scope of skills of a person skilled in the art and depends on the activity of the product according to the invention, place of activity, and innate sensitivity of vertebrates in need of the treatment, such vertebrates including mammals and birds. Alternatively, a therapeutically effective amount is sufficient to cause an improvement in the host's clinically significant condition.

As used herein, 'treatment' means treatment in order to cure, which may be a complete or partial recovery from the condition or states related to gastritis, gastric and duodenal ulcer, intestinal disorders, GIT disorders, and diarrhoea.

As used herein, the term 'alleviation' means the reduction, i.e. less severe or milder condition or states related to gastritis, gastric and duodenal ulcer, intestinal disorders, GIT disorders, and diarrhoea.

As used herein, the term 'prevention' or 'prophylaxis' means a complete or partial inhibition of the development or outbreak of the defined state or states related to gastritis, gastric and duodenal ulcer, intestinal disorders, GIT disorders and diarrhoea. Determination of a preventively effective amount remains within the scope of skills of a skilled artisan, and depends on the activity of the product, place of activity and innate sensitivity of an individual vertebrate in need of such treatment, the vertebrate including a mammal and bird. Alternatively, a preventively effective amount is sufficient to protect the host against the conditions related to gastritis, gastric and duodenal ulcer, intestinal disorders, GIT disorders, and diarrhoea.

*Development of the product:* The presented project concerns the development of a product for use in the treatment, alleviation or prevention of the growth of *H. pylori* and other bacteria in various medical conditions. The conditions which come to mind with respect to the efficiency of the product in the treatment, alleviation or prevention of the growth of *H. pylori* and other bacteria in various medical conditions are, although are not limited to, gastritis and other related diseases, e.g. gastric and duodenal ulcer, intestinal disorders, GIT disorders, and diarrhoea.

Also disclosed is a method for obtaining an improvement in the health status and in functioning of stomach and intestine, and GIT in vertebrates, including mammals and birds, wherein the method comprises administration to vertebrates, including mammals and birds, a sufficient amount and optionally, at a sufficient rate capable of inducing the desired effect, cultures of *L. reuteri* DAN080, partially inactivated cultures of *L. reuteri* DAN080, liquid supernatants of *L. reuteri* DAN080 cultures, concentrated supernatants *of L. reuteri* DAN080 cultures and dried supernatants *of L. reuteri* DAN080 cultures.

The changes improving the health status and functioning of stomach, intestine, and GIT in vertebrates subjected to the treatment are compared with the health status and functioning of stomach, intestine and GIT in vertebrates which are not recipients of the antimicrobial agent of the invention. The changes are considered as an improvement if they are beneficial for the vertebrates in need of such treatment, including mammals and birds.

In other solutions, the above-mentioned method is the method employing *L. reuteri* DAN080 cultures, partially inactivated *L. reuteri* DAN080 cultures, liquid supernatants *of L. reuteri* DAN080 cultures, concentrated supernatants of *L. reuteri* DAN080 cultures and dried supernatants *of L. reuteri* DAN080 cultures.

In further solutions according to the invention, the vertebrates including mammals and birds are, for instance, humans, domestic animals, pets, animals involved in sports, broilers, layer hens, mice, rats, guinea pigs, rabbits and other laboratory animals, including primates, independently of their age.

*Administration of liquid supernatant, condensed supernatant and dried supernatant obtained from the L. reuteri DAN080 culture:* Administration may be accomplished via various routes selected according to the type of vertebrate to be treated, the condition of the vertebrate in need of the treatment by the above described methods, and the specific indication for treatment.

In accordance with one solution, the agent is administered in the form of food or feed additive, such as dietary supplement and/or component in solid form and/or in the form of a beverage. Further solutions may be in the form of suspensions or solutions, such as the beverages further described below. Suitable forms may also be aerosols, globules, suppositories, capsules or tablets, chewable or soluble, e.g. effervescent tablets, as well as powders and other dry forms known to those skilled in the art, such as granules, for example microgranules.

The administration may be parenteral, rectal, intravaginal, inhalatory and oral, in the form of additives to feed or food, as disclosed herein above. Vehicles for parenteral administration include sodium chloride solution, Ringer's solution with dextrose, dextrose and sodium chloride solution, Ringer's solution with lactates or plant oils.

Feed and feed additive may be also emulsified. The therapeutically active component may subsequently be mixed with pharmaceutically acceptable excipients compatible with the active component. Suitable excipients are, for example, water, saline, dextrose, glycerol, ethanol, or the like, and combinations thereof. In addition, if desired, the composition may contain trace amounts of auxiliary substances, such as lubricating or emulsifying agents, pH modulating agents, buffering agents, which enhance the effectiveness of the active component.

Various forms of feeds or feed additives may be provided, such as solid, liquid, lyophilized, or dried otherwise. They may include diluents as for example various buffers (e.g., Tris-HCl, acetate, phosphate buffers) having various pH ranges and ionic strength, additives, such as albumin, gelatin, detergents (e.g. Tween 20, Tween 80, Pluronic F68, bile acid salts), solubilizing agents (e.g. glycerol, polyethyleneglycerol), antioxidants (e.g. ascorbic acid, sodium metabisulfite), preservatives (e.g. thimerosal, benzyl alcohol, parabens), bulking substances or tonicity modifiers (e.g. lactose, mannitol), polymers, such as polyethylene glycol, polymers forming complexes with metal ions, polylactic acid, polyglycolic acid, hydrogels, etc., or liposomes, nanocapsules, microemulsions, micelles, unilamellar or multilamellar vesicles, erythrocyte ghosts, spheroplasts or chitin derivatives.

*Beverages:* In one solution, the feed or feed additive is administered in the form of a beverage, or its dry formulation, by any method disclosed.

The beverage contains an effective amount of the product in the form *of L. reuteri* DAN080 cultures, partially inactivated *L. reuteri* DAN080 cultures, liquid supernatants of *L. reuteri* DAN080 cultures, concentrated supernatants of *L. reuteri* DAN080 cultures, and dried supernatants of *L. reuteri* DAN080 cultures, or mixtures thereof, together with a water-soluble , nutritionally acceptable carrier, such as mineral components, vitamins, carbohydrates, fats and proteins. All such components are supplied in a dried form, if and when the beverage is provided in a dry form. The beverage supplied in the form ready for consumption additionally contains water. The final solution of the beverage may also have a controlled tonicity and acidity, e.g. as a buffered solution, in accordance with the general suggestions provided in the paragraph above.

The pH remains preferably within the range from 2-5, especially 2-4, for the prevention of bacterial and fungal growth. A sterilized beverage may also be used, with pH of approximately 6-8.

The beverage may be supplied alone or in combination with one or more of therapeutically effective compositions.

The use of *L. reuteri* DAN080 cultures, partially inactivated *L. reuteri* DAN080 cultures, liquid supernatants of *L. reuteri* DAN080 cultures, concentrated supernatants of *L. reuteri* DAN080 cultures, and dried supernatants of *L. reuteri* DAN080 cultures and other above-mentioned forms of antimicrobial agent of the present invention for manufacturing a composition for prevention, alleviation or treatment of gastritis and other related diseases, e.g. gastric and duodenal ulcer, intestinal disorders, GIT disorders and diarrhoea.

Further solutions according to the invention cover uses where the composition is a pharmaceutical composition. This pharmaceutical composition may include pharmaceutically acceptable carriers and/or additives, such as diluents, preservatives, solubilizing agents, emulsifiers, adjuvants and/or carriers useful in the methods, the use of which has been disclosed herein in accordance with the invention.

Furthermore, as used herein, 'pharmaceutically acceptable carriers' are well known to skilled artisans and may cover, but are not limited to, 0.01-0.05 M phosphate buffer or 0.8% saline. In addition, such pharmaceutically acceptable carriers may be aqueous or non-aqueous solutions, suspensions and emulsions. Examples of non-aqueous solvents are propylene glycol, polyethylene glycol, plant oils, such as olive oil, and injectable organic esters, such as ethyl oleate. Aqueous carriers include water, alcoholic/aqueous solutions, emulsions or suspensions, including saline and buffered media. Parenteral vehicles include sodium chloride solution, Ringer's solution with dextrose, dextrose and sodium chloride solution, Ringer's solution with lactates or plant oils. Preservatives and other additives may also be present, such as antimicrobials, and antioxidants, chelating agents, inert gases, and the like.

Still further solutions according to the invention cover uses wherein the composition is a dietary supplement and/or a component in the form of solid food and/or beverage. Such a composition of the invention, such as a pharmaceutical composition or that supplied with food or feed, may optionally contain a carrier and/or a certain amount of a second or subsequent active component, exerting an effect on gastritis and other related diseases, e.g. gastric and duodenal ulcer, intestinal disorders, GIT disorders and diarrhoea.

The improvement in and prevention of gastritis and other related diseases, e.g. gastric and duodenal ulcer, intestinal disorders, GIT disorders and diarrhoea, takes place as a result of administration of compositions based on *L. reuteri* DAN080 cultures of, partially inactivated *L. reuteri* DAN080 cultures, liquid supernatants of *L. reuteri* DAN080 cultures, concentrated supernatant of *L. reuteri* DAN080 cultures, and dried supernatants *of L. reuteri* DAN080 cultures, and other above-mentioned forms of the antimicrobial agent of the invention. The therapeutically effective amount is approximately 0.0001 - 0.2 g/kg of body weight per day.

*Target groups for administration:* As will be readily understood by one skilled in the art, the methods and pharmaceutical compositions according to the present invention are particularly suitable for administration to humans, domestic animals, pets, animals involved in sports, broilers, laying hens, mice, rats, guinea pigs, rabbits, and other animals, e.g. laboratory animals, including the primates, wild animals (living in and/or outside zoos), irrespective of age.

Launching of salami sausage or other marketable product is being considered, the product showing special health promoting properties, and being prepared by a the technological process involving lactic fermentation bacteria, making use of the following:
1) bacteria of the genus *Lactobacillus -* especially *L. reuteri* DAN080, showing specific probiotic characteristics;
2) technique which would allow preserving in the product: salami sausage (or others), live bacterial cultures. The products of fermentation of these bacteria ensure an excellent flavour quality of the product, and simultaneously, maintain bacterial homeostasis of the gastrointestinal tract of a consumer.

Due to their specific antibacterial properties, the fermentation products:
- reduce colonization by pathogenic bacteria, including *H. pylori,*
- protect against infections of the gastrointestinal tract,
- alleviate the course of infections, among others *H. pylori* infections
- prolong the expiry date of the product
- when used for coating of sheathing, protect the product against contaminations, while preserving the same.

Generally, the term probiotics refers to such microorganisms which have been commercially used as additives to food and feed, and also have found their use in the pharmaceutical industry. The definition of 'probioticum' comes from the middle 1970s, when selected microbes were used in the feeding of animals.

Probiotics enhance public health conditions, which enhancement results mainly from protection of the population against systemic diseases and infections, and also from weakening of the symptoms or reduction of effects of such systemic diseases. Therefore, studies on probiotics concern the assessment of the scope within which the specific bacterial strains prevent the occurrence of disorders, and also explain the circumstances of their health promoting effect.

The alimentary system, being a highly organized ecosystem, due to protective properties of the intestine microbiota, intestine mucosa and the immune system, provides an effective barrier against pathogenic microorganisms. Probiotics enjoy a great interest in supplementary therapy in the states of bacterial gastro-intestinal infections. At present, intensive research is being conducted in many centers concerning *H. pylori* infections, which are clinically manifested mainly by gastric and duodenal ulcer, and contribute to the development of gastric cancer. Considering the fact of epidemiological spread *of H. pylori* infections in various regions worldwide, it becomes important to find methods which would limit the process.

The health promoting activity of lactic acid bacteria consists mainly of prevention of colonization of the mucous membranes (e.g. of the intestine) by undesirable microbiota, while the products secreted and released by the bacteria to the extracellular surroundings, such products as active compounds: acids, hydrogen peroxide (protons), enzymes, bacteriocins, or bacterial degradation products: fragments of the bacterial wall, affect the growth of other microorganisms (including pathogenic) and thus harmonize the function of the alimentary system. Post-fermentation products of lactic acid bacteria metabolism possess also a capability of decreasing the level of bacterial toxins and micotoxins (fungal metabolites).

The bacteria *L. reuteri* DAN080 are also characterized by the resistance to the effect of thermal stress occurring during the fermentation process. The production of lactic acid, the agent providing products with sensory qualities, should be maintained by bacteria at a relatively constant level, irrespective of the temperature in which the fermentation develops and the product is stored.

The unique bacteria of the genus *Lactobacillus* - *L. reuteri* DAN080, are intended, among other things, to be marketed as a probiotic in the production of cold cuts of meat. The results of preliminary studies with the use of an animal model show that, following an oral administration of the fermentation products of these microorganisms to the infected (with *H. pylori* and other bacteria causing gastrointestinal tract infections) stomach of a mouse, the clear reduction of the infection occurs. The studies show that thermostabile fermentation products of the bacteria *L. reuteri* DAN080 improve the immune condition in infected mice, and at the same time, protect the stomach against further colonization. The observations supported by the *in vitro* studies allow the speculations that a decrease in the distribution *of H. pylori* as a result of the activity of fermentation products of the bacteria *L. reuteri* DAN080, may lead to a similar effect in humans at risk of gastric ulcer, or patients with ulcerative gastritis and duodenitis and other infections of the gastrointestinal tract.

The characteristics of cellular metabolites observed in laboratory conditions enable the assumption that *L. reuteri* DAN080 may be used in the technology used in production of salami sausage and other food products and beverages.

It is anticipated that the life functions of lactic fermentation bacteria *L. reuteri* DAN080 will be maintained after the manufacturing of salami sausage (or other products). It is also anticipated that the innovative fermentation process will increase bioavailability of macro- and microelements from such a salami sausage, primarily of calcium and magnesium. It should be emphasized that the presence of live bacteria will considerably extend durability of the salami sausage (or other products) without the addition of artificial preservative agents.

It should also be indicated that to-date, on the market of food products, despite the existing traditions, there is a lack of production of this type of salami sausage with the ability of harmonizing the function of the intestines and improving the digestion process of consumers.

### EXAMPLES

### Example 1. Effect of fermentation products of L. reuteri DAN080 and other lactic fermentation bacteria on H. pylori colonization in the mouse stomach.

48 mice (BALB/cA) divided into 4 groups of 12 mice each were involved in the study.

The first group of mice were administered daily, by a gastric tube, for 35 days, a preparation 1 (definition 1) - 0.5 ml of a mixture of neutral supernatant obtained from 10-hour culture of *L. reuteri* DAN080 cells in stationary phase, and other lactic acid bacteria, listed in Tables 1-4, having an anti- *H*. pylori activity in combination with calcium alpha-ketoglutarate (30 mM) or chitosan alpha-ketoglutarate, or others, or other alpha-ketoacids salts administered in a liquid form, or contained in bakery products or in crisps. Starting from the 11^{th} day of the experiment, the same mice were administered twice a week for the subsequent 2 weeks, 1 hour after the first treatment a portion of 0.2 ml of fresh microscopically monitored sub-culture *of H. pylori* cells (10⁸ cells/ml) suspended in BHI.

The second group of mice was administered daily via gastric tube, for 35 days preparation 2 (limited definition 2) - 0.2 ml (10⁸ cells/ml) of *L. reuteri* DAN080 and other lactic acid bacteria reported in Tables 1 - 4 cells suspended in MRSB, or administered with bakery products or crisps, exhibiting anti-*H*. *pylori* activity in combination with calcium alpha-ketoglutarate (30 mM) or chitosan alpha-ketoglutarate, or others, or salts of other alpha-ketoacids, and subsequently, starting from the 11^{th} day of the experiment the mice were infected with *H. pylori* according to the infection scheme, as described for the first group.

The third group of mice was administered daily, intragastrically by a gastric tube, for 35 days preparation 3 (definition 3) - cells *of L. reuteri* DAN080 and other lactic acid bacteria, being the property of the inventor (10⁸ cells/ml) suspended in 0.5 ml MRSB, or administered with bakery products or crisps, having an anti- *H. pylori* activity in combination with a mixture of a neutral supernatant obtained from a 10-hour culture *of L. reuteri* DAN080 in stationary phase, and other lactic acid bacteria constituting the property of the inventor, and calcium alpha-ketoglutarate or chitosan alpha-ketoglutarate, or salts of other alpha-ketoacids.

The fourth group (positive control), twice a week for two weeks was fed by gastric tube with 0.2 ml of fresh microscopically monitored sub-culture of *H. pylori* cells (10⁸ cells/ml) suspended in BHI.

The results are shown in Tables 3-5.

On the 36^{th} day, all mice were sacrificed and their stomachs were examined for the presence of *H. pylori* in the mucosa - Table 3.

**Table 3. Presence of H. pylori in gastric mucosa of mice from experimental groups I - IV.**

| Section of gastrointestinal tract | Colonization with *H. pylori* after previous treatment as in: | | | |
|---|---|---|---|---|
| | Group I | Group II | Group III | Group IV |
| Stomach | - | - | - | + |

### Example 2. Effect of fermentation products of L. reuteri DAN080 and other lactic fermentation bacteria on the colonization by ureolytic microbiota, in wild animals.

A diet of a group of wild animals from a zoo (n=10), without clinical symptoms indicating the disruption of the continuity of the gastrointestinal tract epithelium, constantly exposed to stress due to a rapid and permanent change of nutritional and environmental conditions, and therefore exposed to infections with ureolytic bacteria, was supplemented for 60 days, with the preparation 4 (definition 4) - being a mixture of neutral supernatant of a 10-hour culture of the cells of *L. reuteri* DAN080 in a stationary phase and other lactic acid bacteria mentioned in Tables 1- 4, or contained in bakery products or crisps, exhibiting an anti-ureolytic bacteria activity in combination with the cells of *L. reuteri* DAN080 and other lactic acid bacteria constituting the property of the present inventor, exhibiting the activity against ureolytic bacteria, in combination with calcium alpha-ketoglutarate (30mM) or chitosan alpha-ketoglutarate, or others, or salts of other alpha-ketoacids, or in combination with bakery products, crisps.

After the introduction of such additives to the diet/feed, and for a further 30 days of observations, the animals maintained good health and general well-being, with no fever, diarrhoea, or other symptoms of infection occurring.

### Example 3. Effect of fermentation products of L. reuteri DAN080 and other lactic acid bacteria on the colonization of the skin of the back and face of young volunteers, aged 13-17 (n=12), with the diagnosis of Acne vulgaris, by Propionibacterium acnes.

In the first group, the volunteers (n=4) were administered twice a day, for 30 days, a preparation (limited definition 5) made of a mixture of neutral supernatant obtained from a 10-hour culture *of L. reuteri* DAN080 cells in a stationary phase, and supernatants from the cultures of other lactic acid bacteria constituting the property of the present inventor, exhibiting anti-ureolytic bacteria activity with calcium alpha-ketoglutarate or sodium alpha-ketoglutarate or chitosan alpha-ketoglutarate, or others, or with salts of other alpha-ketoacids administered in the form of an ointment or moist compress.

The second group of volunteers (n=4) received twice a day, for 30 days, a preparation (limited definition 6) made of the cells of *L. reuteri* DAN080 and other lactic acid bacteria, mentioned in Tables 1 - 4, exhibiting anti-ureolytic bacteria activity in combination with calcium alpha-ketoglutarate or sodium alpha-ketoglutarate or chitosan alpha-ketoglutarate, or others, or with salts of other alpha-ketoacids in the form of an ointment or moist compress.

The third group of volunteers (n=4) was administered twice a day, for 30 days a preparation (narrowed definition 7) of cells of *L. reuteri* DAN080 and other lactic acid bacteria constituting the property of the present inventor, exhibiting anti-ureolytic bacteria activity in combination with the mixture of neutral supernatant obtained from a 10-hour culture *of L. reuteri* DAN080 in a stationary phase, and other supernatants from cultures of lactic acid bacteria constituting the property of the present inventor, having anti-ureolytic bacteria activity, and calcium alpha-ketoglutarate or sodium alpha-ketoglutarate or chitosan alpha-ketoglutarate, or others, or with salts of other alpha-ketoacids - administered in the form of an ointment or moist compress.

In the course of the study, in all the volunteers, healing was observed of the infected sites where *Acne vulgaris* occurred. No new foci of acne developed in any of the volunteers. During the 15-day observation, after termination of the administration of the preparations, no reinfection was noted.

**Table 8. Presence of P. acnes in the skin of the back and face of the volunteers infected (Groups: I - III).**

| Skin | Colonization by *P. acnes* after previous treatment | | |
|---|---|---|---|
| | Group I | Group II | Group III |
| Back | - | - | - |
| Face | - | - | - |

It was unexpectedly found out that the preparation is effective against chronic, porous fissure-like infections with ureolytic bacteria, including infections of the feet, armpits and groin, and epidermis products, such as nails, hair, hooves and horns. The preparation protects and reduces infections in the form of abscesses and boils, as well as sycosis, exfoliative dermatitis of infants, erysipelas, impetigo contagiosa, ecthyma, folliculitis, *Acne vulgaris,* difficult to treat erythrasma caused by *Propionibacterium minutisimum,* infections of surgical and burn wounds, and bed sores. Due to its activity reducing the colonization of the skin surface and skin products, the unpleasant odour caused by changes in the pH of the skin, and the presence on its surface of odorous extracellular metabolites of ureolytic, bacteria become eliminated.

Lactic acid bacteria constituting the property of the present inventor are easily cultured on liquid or solid media MRS (de Man Rogosa Sharpe) for 24 hours at the temperature of 37°C, in microaerophilic conditions. The above-mentioned strains show characteristics which may be considered as conducive to the colonization of the gastriointestinal tract. They possess a capability of binding matrix proteins, especially collagen and fibronectin, which favours the adhesion to the epithelium of the intestine. These bacteria release proteases causing the decomposition of milk proteins, and fermentation of saccharides contained in milk, which facilitates access of the microorganisms to the nutritional substrate. Besides, for some of them inulin may be the source of carbon. Therefore, while fermenting this indigestible fructan, irrespective of other biochemical activities biased against ureolytic bacteria, the bacteria participates in the regulation of the local intestinal microbiota. All strains survive for one hour in a medium containing 20% of bovine bile, in acidic conditions, in pH of 2.5 for 2 hours, which indicates that after oral administration they may pass intact via the stomach and small intestine to the large intestine. They do not develop resistance to antibiotics and chemotherapeutics to the degree that they would be disqualified as microorganisms potentially settling the gastrointestinal tract in humans and animals.

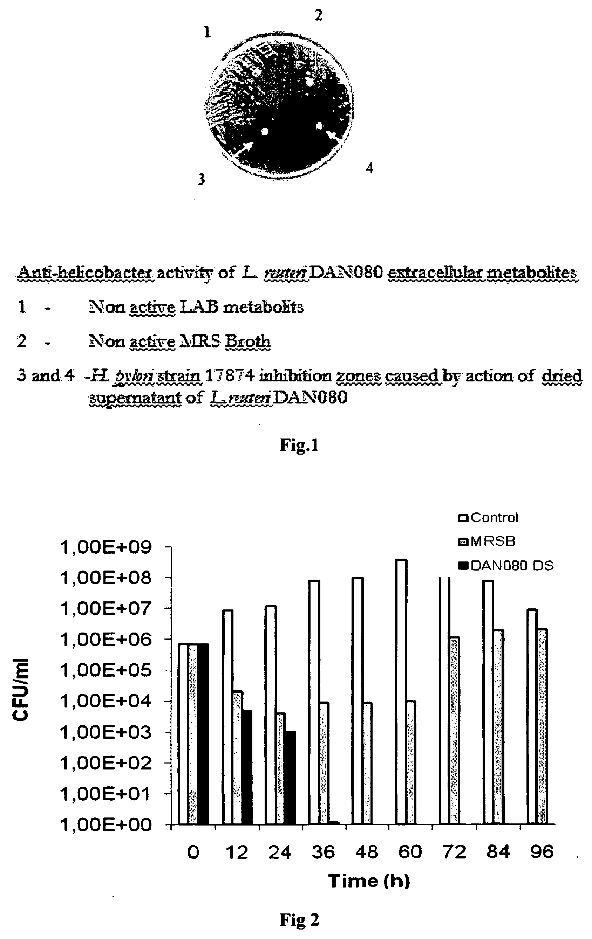

It was confirmed that the following bacteria show activity against ureolytic pathogens of the gastrointestinal tract, urinary tract, body surface, and respiratory system:
- bacteria *L. reuteri* DAN080 with bactericidal effect on *H. pylori* and other pathogens of the gastrointestinal tract by their extracellular metabolism products (Fig. 1).
- other lactic acid bacteria constituting the property of the present inventor, which during their growth maintain the capability of releasing alpha-ketoglutarate into the environment as one of their metabolites. Alpha-ketoglutarate in turn, acting locally, in the appropriate concentrations (30 mM), hydrolyses urea present in the environment, thus interfering with the process of colonization by other bacteria - ureolytic pathogens, the growth of which is dependent of the pH of the microenvironment, and is impossible in, e.g. acid pH of the stomach.

Within the region of the gastric mucosa this phenomenon covers not only such bacteria as *H. pylori,* but also *Proteus mirabilis, Citrobacter freundii, Klebsiella pneumoniae, Enterobacter cloacae, Staphylococcus aureus, Staphylococcus capitis urealiticum* (see: Osaki T et al. Urease-positive bacteria in the stomach induce a false-positive reaction in a urea breath test for diagnosis of Helicobacter pylori infection. J Med Microbiol. 2008; 57:814-9; Brandi G et al. Urease-positive bacteria other than Helicobacter pylori in human gastric juice and mucosa. Am J Gastroenterol. 2006;101(8):1756-61), which use their own urease for urea decomposition, thus providing themselves with habitation microniches.

### Example 4. Activity tests in vivo

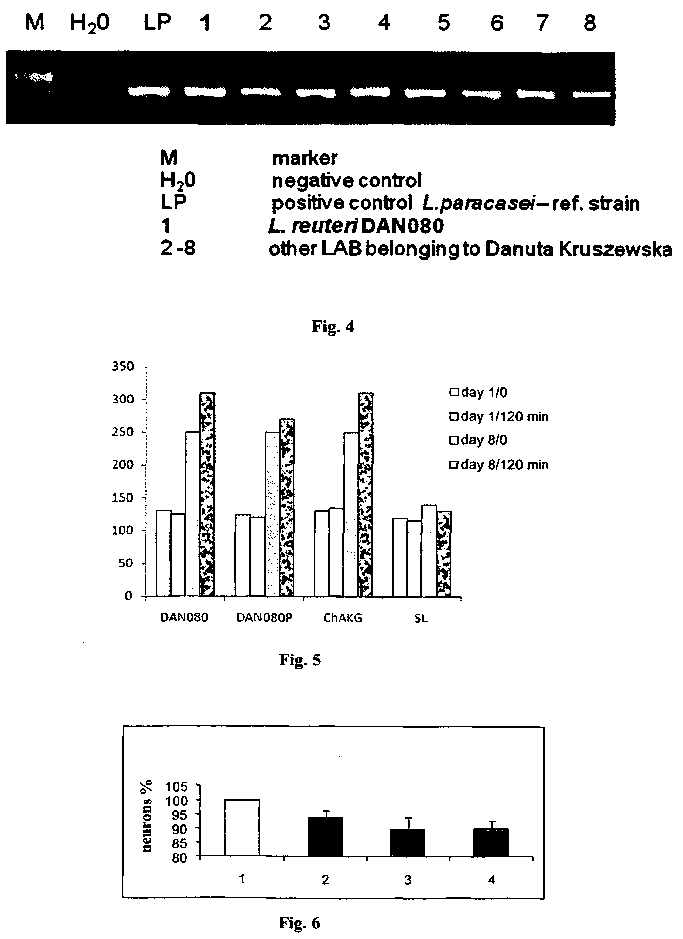

a) Activity of lysozyme: Forty eight 2-month-old Sprague Dawley female rats with a weight of 140-275 g, were fed with feed adequate for the age of the animals, and watered *ad libitum.* Three days prior to experiment, all animals had catheters inserted in the jugular vein. The study was started by taking blood samples from the rats. Subsequently, the animals were administered intragastrically, by means of a gastric tube, 0.5 ml of the following preparations: suspension of the bacteria *L. reuteri* DAN080 - live and dead cells, chitosan AKG suspension, saline. Hundred twenty min. after the first blood taking the second blood sample was taken from the animals. On the second day, the rats received the same preparations once daily for 7 subsequent days. Twelve rats received live bacteria at a dose of 10⁶ cells suspended in physiological saline. The following 12 animals also received for 8 days, 10⁶ each of thermally killed cells *L. reuteri* DAN080. To the next 12 rats chitosan AKG suspension was administered, and the fourth group of animals (n=12) were administered intragastrically for 8 days, at each time 0.5 ml physiological saline. On day 8 after the final dose of the preparations administered intragastrically, blood was taken from the animals from the jugular vein. For the second time on the same day, blood was taken from all rats 120 min. after the first blood taking.
   Determinations of the lysozyme activity in the blood were performed in the presence of a suspension of *Micrococcus lysodeikticus* cells of specified density, based on the absorbance value, and comparing this value with the absorbance curve plotted from a number of standard dilutions of crystal lysozyme (Sigma-Aldrich) in PBS and suspension of *M. lysodeikticus* cells of specified density. After 15, 30, 45, 60 min., incubation absorbance was measured at the wave length of 540 nm.
   Results: A stimulation of rat immune system was observed by live and thermally killed bacteria *L. reuteri* DAN080 and by chitosan AKG. Fig. 5 presents the results.
b) The effect of *L. reuteri* DAN080 metabolites on neurons of the enteric nervous system in swine: Neurons of the enteric nervous system were isolated from the middle section of the small intestine in 3-6 -week old piglets (n=5) with a body weight of 15 kg. The tissues were treated with tripsin, type 2 collagenase and protease in order to obtain neuron cultures. The neuron cultures were plated in Neurobasal A medium for neurons, enriched with an additive of fetal bovine serum, or in the presence of neutralized metabolites of *L. reuteri* DAN080, and twice or four-times concentrated samples of metabolites. The cultures were maintained in an atmosphere of 5% CO₂ at 37°C for 6 days.
   Results: After 6 days of incubation, it was observed in the control samples (neurons cultured on medium) that 53.7 ± 2.7% of cells survived. Considering this fact, the index of neurons was determined which survived in the presence *of L. reuteri* DAN080 metabolites, with relation to the neurons incubated exclusively on the enriched Neurobasal A medium. The cells *of L. reuteri* DAN080 isolated from mouse stomach, through their metabolites, do not statistically significantly decrease the survival rate of neurons isolated from the nervous system in piglets. The cells *of L. reuteri* DAN80 do not cause the degeneration of the nervation (structure) of the gastrointestinal tract. Fig. 6 presents the results.

### SEQUENCE LISTING

<110> EUROCHIT Danuta KRUSZEWSKA,
<120> NEW METHOD FOR OBTAINING NANOPRODUCT USEFUL IN PROPHILAXIS AND MEDICAL AND VETERINARY TREATMENT
   (Nowy sposob uzyskiwania nanoproduktu uzytecznego
   w profilaktyce i medycynie ludzkiej i weterynaryjnej)
<130> I/171300.EP
<140> not known yet
   <141> 2010-11-29
<160> 6
<170> PatentIn version 3.3
<210> 1
   <211> 659
   <212> DNA
   <213> murine
<400> 1 
tggaaacaga tgctaatacc gcataacaac aaaagccaca tggcttttgt
ttgaaagatg gctttagcta tcactctggg atggacctgc ggtgcattag 100
ctagttggta aggtaacggc ttaccaaggc gatgatgcat agccgagttg
agagactgat cggccacaat ggaactgaga cacggtccat actcctacgg 200
gaggcagcag tagggaatct tccacaatgg gcgcaagcct gatggagcaa
caccgcgtga gtgaagaagg gtttcggctc gtaaagctct gttgttggag 300
aagaacgtgc gtgagagtaa ctgttcacgc agtgacggta tccaaccaga
aagtcacggc taactacgtg ccagcagccg cggtaatacg taggtggcaa 400
gcgttatccg gatttattgg gcgtaaagcg agcgcaggcg gttgcttagg
tctgatgtga aagccttcgg cttaaccgaa gaagtgcatc ggaaaccggg 500
ccacttgagt gcagaagagg acagtggaac tccatgtgta gcggtggaat
gcgtagatat atggaagaac accagtggcg aaggcggctg tctggtctgc 600
aactgacgct gaggctcgaa agcatgggta gcgaacagga ttagataccc
tggtagtcc 659
<210> 2
   <211> 22
   <212> primer 1
<400> 2
   TGGAAACAGA TGCTAATACC GC 22
   Reference: Left primer (genus specific): LactoF (Byun R, Nadkarni MA, Chhour KL, Martin FE, Jacques NA, Hunter N. Quantitative analysis of diverse Lactobacillus species present in advanced dental caries. J Clin Microbiol. 2004;42(7):3128-36).
<210> 3
   <211> 20
   <212> primer 2
<400> 3
   ATTAGATACC CTGGTAGTCC 20
   Reference: Right primer 806F (Fredricks DN, Relman DA. Strona 1
   SEQUENCE LISTING improved amplification of microbial DNA from blood cultures by removal of the PCR inhibitor sodium polyanetholesulfonate.
   J Clin Microbiol. 1998; 36(10):2810-6).
<210> 4
   <211> 19
   <212> primer 3
<400> 4
   AGCAGTAGGG AATCTTCCA 19
<210> 5
   <211> 18
   <212> primer 4
<400> 5
   ATTYCACCGC TACACATG 18
<210> 6
   <211> 22
   <212> primer 5
<400> 6
   ACAATGGACG AAAGTCTGAG TG 22
   Reference for primers 3-5 (seg.4-6): walter, J., Hertel, ch., Tannock GW., Lis CM., Munro K., Hammes W.P. (2001) Detection of Lactobacillus, Pediococcus, Leuconostoc i weisella species in human feces by using group specific PCR primers and denaturing gradient gel elektophoresis. Appl. Environ. Microb. 67, 2578-2585

## Claims

1. *Lactobacillus reuteri* DAN080 strain with deposit number DSM 15693 for use in medicine.

2. *Lactobacillus reuteri* DAN080 strain with deposit number DSM 15693 wherein the strain is in form of a culture, a partially inactivated culture, liquid, concentrated and dried supernatant of *L. reuteri* DAN080 with deposit number DSM 15693 culture, for use as an antimicrobial agent in prophylaxis and treatment of medical conditions developing as a result of infections caused by bacteria, fungi, and other pathogens of the gastrointestinal tract, body surface, and other systems, such as urogenital system, respiratory system, in vertebrates.

3. *Lactobacillus reuteri* DAN080 strain with deposit number DSM 15693, wherein the strain is in a form selected from the group comprising the whole *L. reuteri* DAN080 strain with deposit number DSM 15693 culture, liquid, concentrated and dried supernatant obtained from *L. reuteri* DAN080 strain with deposit number DSM 15693 culture and from mixed bacterial cultures containing *L. reuteri* DAN080 strain with deposit number DSM 15693; or their mixtures, for use as an antimicrobial agent in prophylaxis and treatment of medical conditions developing as a result of infections caused by bacteria, fungi, and other pathogens of the gastrointestinal tract, body surface, and other systems, such as urogenital system, respiratory system, in vertebrates.

4. *Lactobacillus reuteri* DAN080 strain with deposit number DSM 15693 for use according to claims 1 to 3, wherein the vertebrate is a human individual.

5. *Lactobacillus reuteri* DAN080 strain with deposit number DSM 15693 for use according to claims 1 to 3, wherein the vertebrate is a domestic animal, pet, animal involved in sport, broiler, layer hen, mouse, rat, guinea pig, rabbit and other laboratory animals, including primates, independent of its age.

6. *Lactobacillus reuteri* DAN080 strain with deposit number DSM 15693 for use according to claims 1 to 3, wherein the microorganism is a pathogenic bacterium or fungus.

7. *Lactobacillus reuteri* DAN080 strain with deposit number DSM 15693 for use according to claim 6, wherein the pathogenic bacterium is *Helicobacter pylori.*

8. A use of *Lactobacillus reuteri* DAN080 strain with deposit number DSM 15693 for manufacturing a composition for treatment, alleviation, or prophylaxis of GIT disorders, gastritis, gastric ulcer, duodenal ulcer, intestinal disorders and diarrhoea in individuals in need of such a treatment, and being vertebrates including humans, other mammals and birds, comprising said *Lactobacillus reuteri* DAN080 strain with deposit number DSM 15693 in an effective amount, providing achieving the desired preventive or therapeutic result.

9. The use according to claim 8, wherein the composition is designed for killing, inhibiting, regulating, and preventing the growth of *H. pylori* and other microorganisms, and for the administration in an effective amount and at a appropriate rate, necessary for obtaining the desired preventive or therapeutic result.

10. The use according to claim 8, wherein the composition is designed for the treatment, alleviation, or prophylaxis of diarrhoea.

11. The use according to claim 10, wherein treatment, alleviation, or prophylaxis of diarrhoea includes elimination or stabilization of *H. pylori* growth.

12. The use according to claims 8 to 11, wherein the composition is a pharmaceutical composition optionally containing other biologically active substances, such as vitamins, especially D and E, in particular in a nanoform, at preventive or therapeutic doses, pharmaceutically acceptable carriers and/or other additives, as well as antimicrobial chitosan salts, especially alpha-ketoglutarate, citrate and lactate.

13. The use according to claim 12, wherein the pharmaceutical composition is in solid form, divided into single doses containing a therapeutically effective amount of said *Lactobacillus reuteri* DAN080 strain with deposit number DSM 15693, in amount from 0.001 to 0.2 g/kg of body weight per day.

14. The use according to claim 13, wherein the pharmaceutical composition is in the form of tablets or capsules.

15. The use according to claim 12, wherein the pharmaceutical composition is in liquid form, divided into single doses containing a therapeutically effective amount of said *Lactobacillus reuteri* DAN080 strain with deposit number DSM 15693, in amount from 0.001 to 0.2 g/kg of body weight per day.

16. The use according to claim 15, wherein the pharmaceutical composition is in liquid form designed for use as aerosol, cataplasm or moist compress.

17. The use according to claims 8 to 11, wherein the composition is a dietary supplement, food or feed additive.

18. The use according to claim 17, wherein the dietary supplement, food or feed additive is in a solid form and/or in the form of a beverage.

19. The use according to claims 17 to 18, wherein the therapeutically effective amount ranges from 0.001 to 0.2 g/kg of body weight per day.

## Patentansprüche

1. Der unter der Nummer DSM 15693 deponierte Stamm *Lactobacillus reuteri* DAN080 für Anwendung in Medizin.

2. Der unter der Nummer DSM 15693 deponierte Stamm *Lactobacillus reuteri* DAN080, wobei der Stamm eine Form von Kultur, zum Teil von inaktivierter Kultur, des flüssigen, konzentrierten und getrockneten Flüssigkeitsüberstandes der Kultur des unter der Nummer DSM 15693 deponierten Stammes *L. reuteri* DAN080, hat, für Anwendung als antimikrobielles Mittel für Prophylaxe und Behandlung von medizinischen Zuständen, die infolge der durch Bakterien, Pilze und andere Pathogene des Verdauungskanals, der Körperhüllen und anderen Systeme, wie urogenital System und Atmungssystem der Wirbeltieren, bedingten Infektionen, sich entwickelt haben.

3. Der unter der Nummer DSM 15693 deponierte Stamm *Lactobacillus reuteri* DAN080, wobei der Stamm eine Form hat, die aus der Gruppe, die die komplette Kultur des unter der Nummer DSM 15693 deponierten Stammes *L. reuteri* DAN080, den flüssigen, konzentrierten und getrockneten Flüssigkeitsüberstand, der aus der Kultur des deponierten unter der Nummer DSM 15693 Stammes *L. reuteri* DAN080 und aus gemischten bakteriellen Kulturen des unter der Nummer DSM 15693 deponierten Stammes *L. reuteri* DAN080 oder aus ihrem Gemisch erhalten wurde, umfasst, ausgewählt ist, für Anwendung als antimikrobielles Mittel für Prophylaxe und Behandlung von medizinischen Zuständen, die infolge der durch Bakterien, Pilze und andere Pathogene des Verdauungskanals, der Körperhüllen und anderen Systeme, wie urogenital System und Atmungssystem der Wirbeltieren, bedingten Infektionen, sich entwickelt haben.

4. Der unter der Nummer DSM 15693 deponierte Stamm *Lactobacillus reuteri* DAN080 für Anwendung nach Ansprüche 1 - 3, wobei das Wirbeltier Mensch ist.

5. Der unter der Nummer DSM 15693 deponierte Stamm *Lactobacillus reuteri* DAN080 für Anwendung nach Ansprüchen 1 - 3, wobei das Wirbeltier ein Haushalttier, ein Begleittier, ein Sportstier, ein (Grill-)Hähnchen, eine Leghenne, eine Maus, eine Ratte, ein Meerschweinchen, ein Kaninchen und andere Versuchstiere, einschl. Primate, unabhängig von deren Alter, ist.

6. Der unter der Nummer DSM 15693 deponierte Stamm *Lactobacillus reuteri* DAN080 für Anwendung nach Ansprüchen 1 - 3, wobei der Mikroorganismus ein pathogenes Bakterium oder Pilz ist.

7. Der unter der Nummer DSM 15693 deponierte Stamm *Lactobacillus reuteri* DAN080 für Anwendung nach Anspruch 5, wobei das pathogene Bakterium *Helicobacter pylori* ist.

8. Verwendung des unter der Nummer DSM 15693 deponierten Stammes *Lactobacillus reuteri* DAN080 für Herstellung der Formulierung, die für Behandlung, Linderung oder Prophylaxe von GIT-Störungen, Gastritis, Magengeschwür, Zwölffingerdarmgeschwür, Darmstörungen und Diarrhoe bei den benötigenden das Individuellen, die Wirbeltiere sind, einschl. Menschen, andere Säugetiere und Vögel, die der genannte unter der Nummer DSM 15693 deponierte Stamm *L. reuteri* DAN080 in einer den erwarteten prophylaktischen oder therapeutischen Effekt sichernden effektiven Menge, enthält.

9. Verwendung nach Anspruch 8, wobei diese Formulierung für Tötung, Inhibierung, Regulierung, Vorbeugung des Wachsens von *H. pylori* und anderen Mikroorganismen und für Verabreichung in einer effektiver Menge und mit geeigneter Geschwindigkeit, die für Erreichung von erwartetem prophylaktischem oder therapeutischem Effekt notwendig ist.

10. Verwendung nach Anspruch 8, wobei diese Formulierung für Behandlung, Linderung oder Prophylaxe von Diarrhoe bestimmt ist.

11. Verwendung nach Anspruch 10, wobei Behandlung, Linderung oder Prophylaxe von Diarrhoe Eliminierung oder Stabilisierung des Wachsens von *H. pylori* umfasst.

12. Verwendung nach Ansprüchen von 8 bis 11, wobei die Formulierung eine pharmazeutische Formulierung ist, die ggf. andere biologisch aktive Substanze, wie Vitamine, besonders D und E, besonders in Nanoform, in prophylaktischen oder therapeutischen Dosen, pharmazeutisch akzeptable Träger und/oder Additive, sowie antimikrobielle Salze von Chitosan, besonders alpha-Ketoglutarat, Zitrat und Laktat, enthält.

13. Verwendung nach Anspruch 12, wobei die pharmazeutische Formulierung eine solide Form hat und auf therapeutisch wirksame Menge von der genannten unter der Nummer DSM 15693 deponierten Stamm *Lactobacillus reuteri* DAN080, in einer Menge von 0,001 bis 0,2 g/kg Körpergewicht pro Tag, enthaltende Einzeldosen aufgeteilt ist.

14. Verwendung nach Anspruch 13, wo die pharmazeutische Formulierung eine Form von Tablette oder Kapsel hat.

15. Verwendung nach Anspruch 12, wobei die pharmazeutische Formulierung eine flüssige Form hat und auf therapeutisch wirksame Menge von dem genannten unter der Nummer DSM 15693 deponierten Stamm *Lactobacillus reuteri* DAN080, in einer Menge von 0,001 bis 0,2 g/kg Körpergewicht pro Tag, enthaltende Einzeldosen aufgeteilt ist.

16. Verwendung nach Anspruch 15, wobei die pharmazeutische Formulierung eine flüssige Form hat, die für Anwendung als Aerosol, Kataplasms oder feuchte Kompresse bestimmt ist.

17. Verwendung nach Ansprüchen von 8 bis 11, wobei die Formulierung ein Diätsupplement, Nahrung oder Futterzusatz ist.

18. Verwendung nach Anspruch 17, wobei das Diätsupplement, Nahrung oder Futterzusatz in solider Form und/oder in Form von Getränk ist.

19. Verwendung nach Ansprüchen von 17 bis 18, wobei die therapeutisch wirksame Menge zwischen 0,001 und 0,2 g/kg Körpergewicht pro Tag liegt.

## Revendications

1. La souche *Lactobacillus reuteri* DAN080, déposée sous le numéro DSM 15693 utile en médecine.

2. La souche *Lactobaccilus reuteri* DAN080, déposée sous le numéro DSM 15693, où cette souche est en forme de la culture, partiellement de la culture inactivée, d'un supernatant liquide, concentré et séchéde la souche *L. reuteri* DAN080, déposée sous le numéro DSM 15693, pour utiliser en caractère d'un antimicrobien dans la prophylaxie et dans le traitement des états médicaux se développant en résultat des infections provoquées par les bactéries, champignons et les autres pathogènes du tube gastro-intestinal, des enveloppes du corps et d'autres systèmes comme le système urogénital, le système respiratoire chez les vertébrés.

3. La souche *Lactobaccilus reuteri* DAN080, déposée sous le numéro DSM 15693, où cette souche est en forme séléctionnée du groupe comprenant la souche *Lactobaccilus reuteri* DAN080 complète déposée sous le numéro DSM 15693, d'un supernatant liquide, concentré et séché obtenu de la souche *L. reuteri* DAN080, déposée sous le numéro DSM 15693 et des cultures bactériennes mixtes, contenant la souche *L. reuteri* DAN080 déposée sous le numéro DSM 15693 ou ses mélanges, pour utiliser en caractère d'un antimicrobien dans la prophylaxie et dans le traitement des états méducaux se développant en résultat des infections provoquées par les bactéries, champignons et les autres pathogènes du tube gastro-intestinal, des enveloppes du corps et d'autres systèmes comme le système urogénital, le système respiratoire chez les vertébrés.

4. La souche *Lactobacillus reuteri* DAN080, déposée sous le numéro DSM 15693 pour l'utilisation selon les revendications 1 à 3, où un vertébré est l'homme.

5. La souche *Lactobacillus reuteri* DAN080, déposée sous le numéro DSM 15693, pour l'utilisation selon les revendications 1 à 3, où un vertébré est l'animal doméstique, l'animal-compagnon, l'animal utilisé dans le sport, la volaille de chair, la poule pondeuse, le souris, le rat, le cobaye, le lapin et les autres animaux de laboratoire, y compris les primates, indépendamment de leur âge.

6. La souche *Lactobacillus reuteri* DAN080, déposée sous le numéro DSM 15693, pour l'utilisation selon les revendications 1 à 3, où un microbe est la bactérie pathogène ou le champignon.

7. La souche *Lactobacillus reuteri* DAN080, déposée sous le numéro DSM 15693, pour l'utilisation selon la revendication 5, où la bactérie pathogène est *Helicobacter pylori.*

8. L'utilisation de la *Lactobacillus reuteri* DAN080, déposée sous le numéro DSM 15693, pour la production de la composition destinée au traitement médical, au soulagement ou à la prophylaxie des troubles GIT, de l'inflammation de l'estomac, de l'ulcère gastric, de l'ulcère duodénal, des troubles de l'intestin et de la diarrhée chez les individus ayant besoin de cela, étant les vertébrés y compris l'homme, les autres mammifères et les oiseaux, comprenant la souche *L. reuteri* DAN080, déposée sous le numéro DSM 15693, en quantité assurant la manière effective pour l'obtention d'un effet préventif ou thérapeutique souhaitable.

9. L'utilisation selon la revendication, où la composition est destinée à tuer, inhiber, régulariser, prévenir l'accroissement de *H. pylori* et d'autres micro-organismes et à appliquer en quantité effective et en rythme approprié, nécessaire pour l'obtention d'un effet préventif ou therapeutique souhaitable.

10. L'utilisation selon la revendication 8, où cette composition est destinée au traitement médical, au soulegement ou à la prophylaxie de la diarrhée.

11. L'utilisation selon la revendication 10, où le traitement médical, le soulagement ou la prophylaxie de la diarrhée comprend l'élimination ou la stabilisation de l'accroissement de *H. pylori.*

12. L'utilisation selon les revendications 8 à 11, où cette composition est une composition pharmaceutique, comprenant éventuellement les autres substances biologiquement actives, telles comme les vitamines, spécialement D et E, et notamment en nanoformes, dans les doses préventives ou curatives, les supports pharmaceutiques admissibles et/ou les additifs, ainsi que les sels de chitosan antimicrobiens, notamment l'alphacétoglutarate, le citrate et le lactate.

13. L'utilisation selon la revendication 12, où cette composition pharmaceutique est en forme solide et est divisées en doses particulières comprenant la quantité therapeutiquement effective de la souche *Lactobacillus reuteri* DAN080 mentionnée, déposée sous le numéro DSM 15693, en quantité de 0,001 à 0,2 g/kg du poids de corps pendant 24 heures.

14. L'utilisation selon la revendication 13, où cette composition est sous forme d'une pilule ou capsule.

15. L'utilisation selon la revendication 12, où cette composition pharmaceutique est sous forme liquide et est divisée en doses particulières comprenant la quantité therapeutiquement effective de la souche *Lactobacillus reuteri* DAN080 mentionnée, déposée sous le numéro DSM 15693, en quantité de 0,001 à 0,2 g/kg du poids de corps pendant 24 heures.

16. L'utilisation selon la revendication 15, où cette composition pharmaceutique est sous forme liquide destinée à appliquer en atomiseur, sous forme des cataplasmes ou compresses humides.

17. L'utilisation selon les revendications de 8 à 11, où cette composition est un supplément de diète, un aliment ou un additif aux fourrages.

18. L'utilisation selon la revendication 17, où un supplément de diète, un aliment ou un additif aux fourrages est sous forme solide et/ou sous forme de boisson.

19. L'utilisation selon les revendications 17 à 18, où la quantité therapeutiquement effective se trouve dans les limites de 0,001 à 0,2 g/kg du poids de corps pendant 24 heures.
